# EUROPEAN PATENT APPLICATION

(11) **EP 2 030 610 A1**
(43) Date of publication of application: **04.03.2009**
(21) Application number: 07253433.2
(22) Date of filing: 31.08.2007
(51) Int. Cl.: A61K 9/00, A61K 9/08, A61K 31/55, A61K 31/5517, A61K 47/10, A61K 47/14, A61K 47/22

(54) **Non-aqueous pharmaceutical compositions**

(71) Applicant: Archimedes Development Limited, Nottingham NG7 2TN (GB)
(72) Inventor: Watts, Peter James, Nottingham NG7 2TN (GB); Smith, Alan, Nottingham NG7 2TN (GB); Castile, Jonathan, Nottingham NG7 2TN (GB); Cheng, Yu-Hui, Nottingham NG7 2TN (GB)
(74) Representative: Crowhurst, Charlotte Waveney

(57) **Abstract**

The present invention provides a composition for intranasal delivery of a drug comprising:
(i) the drug; and
(ii) a non-aqueous vehicle comprising (a) propylene glycol and at least one additional solvent selected from N-methylpyrrolidone, propylene carbonate and at least one propylene glycol fatty acid ester or (b) from about 40 to 100 % by volume of N-methylpyrrolidone.

## Description

This invention relates to pharmaceutical compositions for the nasal administration of poorly soluble drug compounds in which the drug is dissolved in a non-aqueous liquid vehicle.

The nasal route of drug delivery can afford rapid absorption of drugs into the blood circulation. In some cases absorption of almost the whole dose can be achieved and the pharmacokinetics can be similar to those achieved for intravenous administration. Such rapid and effective drug delivery can be useful in the treatment of crisis situations such as pain (including breakthrough pain and trauma pain), migraine, anxiety, convulsions, impotence and nausea.

Generally, it is preferable that compositions for the intranasal delivery of drugs are in the form of an aqueous solution. This is due to ease of manufacture, ease of delivery and good patient acceptability. However, it is not always feasible to formulate a drug as an aqueous solution, for example if the solubility of the drug in aqueous media is inadequate. In such circumstances, one option would be to formulate the composition as a non-aqueous solution utilising solvents in which the drug has higher solubility.

However, the nasal mucosal membrane is a delicate tissue and a non-aqueous vehicle needs to be carefully chosen in order to be acceptable to the patient. In this regard, the ideal vehicle will be odourless, tasteless and free from irritation when applied to the nasal cavity. Nasal solutions are typically delivered from spray devices that may comprise a range of glass, plastic, elastomeric and metal components. It is therefore essential that the vehicle does not interact with components of the spray device and impair the device performance, for example through sorption into plastic or elastomeric parts. It is also important that the characteristics of the liquid are such that it is atomised to form a dispersion of droplets when dispensed using a nasal spray device.

The non-aqueous vehicles described in this application are suitable for producing compositions for the intranasal delivery of a wide range of drug compounds. It will be a straightforward matter for one skilled in the art to determine whether a particular non-aqueous vehicle is suitable for use in combination with a particular drug on the basis of the teaching in this application. For example, this can be done by measuring the solubility of the drug compound in the vehicle. The solubility can be tested by adding an excess of the drug to the vehicle and stirring the mixture for 24 hours at room temperature. Undissolved drug is then removed by filtration or centrifugation and the solution is assayed for dissolved drug content by an appropriate analytical method, such as high performance liquid chromatography.

Therapeutic agents (drug compounds) suitable for use in this invention include, but are not limited to, antibiotics and antimicrobial agents, such as tetracycline hydrochloride, leucomycin, penicillin, penicillin derivatives, erythromycin, sulphathiazole and nitrofurazone; antimigrane compounds, such as naratriptan, sumatriptan, zolmitriptan, rizatriptan, eletriptan, frovatriptan, alnitidan, avitriptan, almotriptan or other 5-HT1 agonists; vasoconstrictors, such as phenylephedrine hydrochloride, tetrahydrozoline hydrochloride, naphazoline nitrate, oxymetazoline hydrochloride and tramazoline hydrochloride; cardiotonics, such as digitalis and digoxin; vasodilators, such as nitroglycerin and papaverine hydrochloride; bone metabolism controlling agents, such as vitamin D and active vitamin D3; sex hormones; hypotensives; anti-tumour agents; steroidal anti-inflammatory agents, such as hydrocortisone, prednisone, fluticasone, prednisolone, triamcinolone, triamcinolone acetonide, dexamethasone, betamethasone, beclomethasone and beclomethasone dipropionate; non-steriodal anti-inflammatory drugs, such as acetaminophen, aspirin, aminopyrine, phenylbutazone, mefenamic acid, ibuprofen, diclofenac sodium, indomethacin, colchicines and probenecid; enzymatic anti-inflammatory agents, such as chymotrypsin and bromelain seratiopeptidase; antihistaminic agents, such as diphenhydramine hydrochloride, chlorpheniramine maleate and clemastine; anti-tussive expectorants, such as codeine phosphate and isoproterenol hydrochloride; analgesics such as opioids (like diamorphine, hydromorphone, buprenorphine, fentanyl, oxycodone, codeine, morphine and its polar metabolites, such as morphine-6-glucuronides and morphine-3-sulphate), or combinations of opioids and other analgesic agents such as non-steriodal anti-inflammatory drugs; anti-emetics, such as metoclopramide, ondansetron, chlorpromazine; benzodiazepines including those suitable for treatment of epilepsy; drugs for treatment of sleeping disorders, such as melatonin; drugs for treatment of asthma, such as salbutamol; drugs for treatment of erectile dysfunction such as apomorphine, sildenafil and alprostadil.

A further class of drug compounds of interest for nasal delivery is the benzodiazepines. These lipophilic drugs act on the central nervous system to cause sedation, hypnosis, decreased anxiety, muscle relaxation, anterograde amnesia and anticonvulsant actions and are widely used in medicine. Conditions which they can be used to treat include epilepsy, insomnia, alcohol dependence, muscular disorders and mania. These drugs can also be used in premedication procedures and in veterinary practice. Examples of benzodiazepine drugs include, but are not limited to, alprazolam, chlordiazepoxide, clonazepam, clorazepate, diazepam, estazolam, flurazepam, halazepam, lorazepam, midazolam, nitrazepam, oxazepam, prazepam, quazepam, temazapem, bromazepam, flunitrazepam and triazolam, bentazepam, brotizolam, clotiazepam, delorazepam, ethyl loflazepate, etizolam, fludiazepam, ketozolam, loprazolam, lormetazepam, nordazepam, mexazolam, nimetazepam, pinazepam and tetrazepam. The structures of some of these benzodiazepines can be found in Goodman and Gilman's The Pharmacological Basis of Therapeutics, 9th edition, McGraw Hill (1996), page 383.

This invention can be applied to any of the classes of drugs and to the specific drugs listed above. In particular, the invention can be applied to any benzodiazepine compound, in particular any of the benzodiazepine drugs listed above. A preferred group of benzodiazepine drugs for use in this invention are diazepam (7-chloro-1,3-dihydro-1-methyl-5-phenyl-2H-1,4-benzodiazepin-2-one), lorazepam (7-chloro-5-(2-chlorophenyl)-1,3-dihydro-3-hydroxy-2H-1,4-benzodiazepin-2-one) and midazolam (8-chloro-6-(2-fluorophenyl)-1-methyl-4H-imidazo[1,5-a][1,4]benzodiazepine).

Lau and Slattery (Int. J. Pharm., 54, 171-174, 1989) investigated the intranasal delivery of diazepam and lorazepam using seven non-aqueous vehicles. These were triacetin, dimethyl sulfoxide, polyethylene glycol 400, Cremophor EL, laureth-9-(polyoxyethylene-9 lauryl ether), isopropyl adipate and azone 1-dodecylazacycloheptane-2-one.

US 5,693,608 describes compositions for intranasal administration comprising an n-ethylene glycol (e.g. polyethylene glycol (PEG)). Examples are provided for diazepam, flunitrazepam and lorazepam dissolved in PEG 400 and flunitrazepam dissolved in a mixture of PEG 400 and glycofurol.

A lorazepam solution for intranasal administration using a solvent carrier comprising polyethylene glycol and propylene glycol is described in US-B-6,610,271.

Supersaturated diazepam solutions are described in WO 2006/122217. Diazepam was dissolved in glycofurol to form a concentrated solution and water was added just prior to administration to form a supersaturated solution. It is claimed in this document that the water improves the nasal acceptability of the formulation. However, the need to add water prior to administration adds to the dosing complexity.

The listing or discussion of an apparently prior-published document in this specification should not necessarily be taken as an acknowledgement that the document is part of the state of the art or is common general knowledge The present inventors have surprisingly found that certain non-aqueous vehicles are suitable for use in compositions for the intranasal delivery of a variety of drug compounds.

The present invention provides compositions for intranasal delivery of a drug comprising (i) the drug, and (ii) a non-aqueous vehicle comprising (a) propylene glycol and at least one additional solvent selected from N-methylpyrrolidone, propylene carbonate and propylene glycol fatty acid esters, or (b) from about 40 to 100 % by volume of N-methylpyrrolidone. Unless otherwise stated, these compositions will be referred to hereinafter as the compositions of the invention and the non-aqueous vehicle will be referred to hereinafter as the vehicle of the invention.

The compositions of the invention may (i) be more stable than, (ii) be better tolerated than, (iii) be less toxic than, (iv) have better pharmacokinetic properties than, (v) be more easily prepared than, and/or (vi) have other useful properties over, compositions known in the prior art. In particular, the compositions of the invention may have one or more of the following advantages:
(a) they contain high concentrations of drug (e.g. higher concentrations than in equivalent prior art compositions);
(b) they can be atomised using a conventional intranasal spray device;
(c) they are well tolerated when applied into the nasal cavity;
(d) they provide a medium in which the drug is chemically stable; and/or
(e) they provide for rapid and efficient intranasal absorption of the drug.

Compositions described herein as being "well tolerated" include those that cause little or no discomfort when applied into the nasal cavity. A "well tolerated" composition is also one that may cause some irritation and/or stinging when applied into the nasal cavity but it is such that the patient is not dissuaded from being administered further doses of the composition. In this respect, the tolerability of a nasal composition may be assessed by methods known to those skilled in the art, for example by use of a questionnaire, such as described in US 5,963,508.

Compositions according to the invention that contain high concentrations of drug have the further advantage that a therapeutic dose of drug can be administered in a very small dose volume. This further improves patient acceptability and tolerance, since if a large volume of liquid is administered into the nasal cavity some of this may drip out of the nostrils. For example, if the dose of drug to be delivered is 5 mg, this will require a dose volume of 0.5 mL for a composition containing 10 mg/mL of the drug compound. If the drug content is increased to 100 mg/mL (for example by use of a composition according to the invention), the dose volume will be reduced to only 0.1 mL.

Moreover, compositions according to the invention also have the advantage that they may be prepared using established pharmaceutical processing methods and employ materials that are approved for use in food or pharmaceuticals or are of like regulatory status.

In one aspect, the invention provides a non-aqueous delivery vehicle comprising propylene glycol and at least one additional solvent selected from N-methylpyrrolidone (1-methyl-2-pyrrolidone), propylene carbonate (4-methyl-2-oxo-1,3-dioxolane) and at least one propylene glycol fatty acid ester. This vehicle will be referred to hereinafter as the "propylene glycol vehicle".

The use of a liquid vehicle comprising propylene glycol and one or more of N-methylpyrrolidone, propylene carbonate and at least one propylene glycol fatty acid ester for intranasal delivery of drugs has not been described before. The use of this liquid vehicle will be discussed below with reference to use in combination with benzodiazepine drugs. However, this is by way of example only and this vehicle may also be used with other drugs such as those listed earlier in this text.

Propylene glycol (also known as 1,2-dihydroxypropane, 2-hydroxypropanol, methyl ethylene glycol, methyl glycol or propane-1,2-diol) is widely used as a solvent in parenteral and non-parenteral pharmaceutical formulations. It is well tolerated when applied to mucosal membranes. However, it is not a good solvent for all drugs and particularly not for all benzodiazepine drugs. Additionally, its viscosity and surface tension make it difficult to atomise using conventional intranasal spray devices.

We have surprisingly found that mixtures of propylene glycol and other specific materials, particularly at least one of N-methylpyrrolidone, propylene carbonate and at least one propylene glycol fatty acid ester, enable stable solutions to be prepared containing high concentrations of drugs, such as benzodiazepines, and which can be successfully delivered using nasal spray devices.

The propylene glycol fatty acid esters used in the present invention may be mono or diesters of propylene glycol and have the basic structure

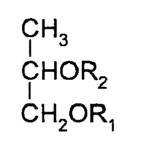

In the case of mono-esters one of R₁ and R₂ is hydrogen and the other is a fatty acid moiety. In the case of diesters R₁ and R₂ are both fatty acid moieties.

In the propylene glycol fatty acid esters used in the present invention, when R₁ and/or R₂ is a fatty acid moiety, they each individually have a carbon chain length which is primarily in the range of from C6 to C18. In other words, when R₁ and/or R₂ is a fatty acid moiety, the propylene glycol fatty acid ester typically is a mixture of esters with different chain lengths (such that primarily R₁ and/or R₂ = C6 to C18 fatty acid moiety).

An especially preferred propylene glycol fatty acid ester for use in this invention is a mono ester of medium chain fatty acids, primarily caprylic acid (C8).

By "primarily", we mean that at least 80% of the fatty acid content of the propylene glycol fatty acid ester is of the type specified.

A propylene glycol fatty acid ester consisting primarily of the monoester of caprylic acid may be described as propylene glycol monocaprylate. Commercial suppliers of propylene glycol monocaprylate include Abitec Inc. (Columbus, Ohio USA) under the trade name Capmul® PG8 and Gattefosse (Saint Priest, France) under the trade names Capryol^{™} 90 and Capryol^{™} PGMC.

The propylene glycol vehicle of the present invention may comprise at least one additional solvent from N-methyl pyrrolidone, propylene carbonate and propylene glycol fatty acid esters. Any combination may be used, for example each of these compounds may be used in a single vehicle. A single propylene glycol fatty acid ester may be used or a mixture propylene glycol fatty acid esters may be used alone or in combination with N-methyl pyrrolidone and/or propylene carbonate.

A particularly preferred combination for use in the present invention is propylene carbonate and a propylene glycol fatty acid ester, for example propylene carbonate and propylene glycol monocaprylate. It has surprisingly been found that the use of propylene carbonate and a propylene glycol fatty acid ester with propylene glycol has a cosolvent effect in that the solubility of a drug compound in a mixture comprising the two additional solvents is greater than the solubility in a solvent comprising one or other of them.

Examples of preferred vehicle compositions (% v/v) are provided in Table 1. The percentages represent the theoretical amount by volume in the final vehicle and do not take into account any non-additive volume changes when the individual components are mixed i.e. in the event that the mixture does not behave as an ideal solution. For example, a vehicle described as comprising 50% v/v propylene glycol and 50% v/v propylene carbonate may be prepared by mixing together 10 ml of each solvent (although the final volume may not necessarily be 20 ml). The composition of a vehicle may also be expressed in % w/w terms. For example, 10 ml of propylene glycol and 10 ml of propylene carbonate weigh approximately 10.37 g and 12.00 g respectively at room temperature. Hence, the final composition of this mixture will be 46% w/w propylene glycol and 54% w/w propylene carbonate.

As a further example, a vehicle comprising 50% w/w propylene glycol and 50% w/w N-methylpyrrolidone may be used to prepare a solution containing 10% w/w of drug compound. The final drug solution will contain 10% w/w drug, 45% w/w propylene glycol and 45% w/w N-methylpyrrolidone.

**Table 1. Preferred nasal delivery vehicles**

| | Composition (% v/v*) | | |
|---|---|---|---|
| | Preferred | More preferred | Most preferred |
| *Composition A* | | | |
| Propylene glycol | 20-95 | 25-90 | 30-85 |
| Propylene carbonate | 5-80 | 10-75 | 15-70 |
| *Composition B* | | | |
| Propylene glycol | 20-95 | 25-90 | 30-85 |
| Propylene glycol FAE** | 5-80 | 10-75 | 15-70 |
| *Composition* C | | | |
| Propylene glycol | 20-95 | 25-90 | 30-85 |
| N-methylpyrrolidone | 5-80 | 10-75 | 15-70 |
| *Composition D* | | | |
| Propylene glycol | 15-80 | 20-75 | 25-70 |
| Propylene glycol FAE | 6-65 | 9-60 | 12-55 |
| Propylene carbonate | 3-55 | 4-50 | 5-45 |
| *Composition E* | | | |
| Propylene glycol | 25-75 | 30-70 | 35-65 |
| Propylene glycol FAE | 10-50 | 15-345 | 20-40 |
| N-methylpyrrolidone | 20-40 | 15-35 | 10-30 |
| *Composition F* | | | |
| Propylene glycol | 30-70 | 35-65 | 40-60 |
| Propylene glycol FAE | 4-40 | 7-35 | 10-30 |
| Propylene carbonate | 4-40 | 7-35 | 10-30 |
| N-methylpyrrolidone | 1-24 | 3-21 | 5-18 |

| | | | |
|---|---|---|---|
| *Theoretical composition of vehicle assuming final volume is equal to sum of volume of individual components **Propylene glycol fatty acid ester e.g. propylene glycol monocaprylate | | | |

The amounts of propylene glycol fatty acid ester in this Table (and in Table 2) are the total amounts of that component, which may be made up with smaller amounts of two or more propylene glycol fatty acid esters.

The drug content of the final compositions, produced by dissolving the drug in the vehicle, is dependent primarily on the dose that needs to be delivered to the patient (i.e. the amount required to give a therapeutic effect), but is preferably from about 0.1 to about 2000 mg/ml, more preferably from about 0.5 to 1500 mg/ml and most preferably from about 1 to about 1000 mg/ml.

In addition to the drug, other ingredients may also be added to the non-aqueous vehicle. These additional ingredients include antioxidants, chelating agents, preservatives, flavourings, sweeteners or other agents generally used in pharmaceutical liquid preparations and well known to those skilled in the art.

Where the drug is a benzodiazepine, the composition is preferably comprises from about 0.1 to 300 mg/ml, more preferably from about 0.5 to 250 mg/ml and most preferably from about 1 to about 200 mg/ml of the benzodiazepine. For example, the preferred midazolam concentration is from about 1 to about 100 mg/ml and the preferred lorazepam concentration is from about 0.5 to about 50 mg/ml.

An especially preferred benzodiazepine compound is diazepam. The concentration of diazepam is preferably from about 1 to about 200 mg/ml, more preferably from about 10 to about 180 mg/ml and most preferably from about 20 to about 160 mg/ml, for example from about 50 to about 150 mg/ml.

The compositions of further preferred nasal delivery vehicles are shown in Table 2 below. These nasal delivery vehicles may be used, for example, when the drug is a benzodiazepine (e.g. diazepam). For the avoidance of doubt, however, it should be understood that the delivery vehicles of the invention may have a composition represented by a combination of the preferred, more preferred and most preferred values of the compositions set out in Table 1 and/or Table 2.

**Table 2. Preferred nasal delivery vehicles**

| | Composition (% v/v*) | | |
|---|---|---|---|
| | Preferred | More preferred | Most preferred |
| *Composition I* | | | |
| Propylene glycol | 30-90 | 40-85 | 45-80 |
| Propylene carbonate | 10-70 | 15-60 | 20-55 |
| *Composition II* | | | |
| Propylene glycol | 35-90 | 45-85 | 50-80 |
| N-methylpyrrolidone | 10-65 | 15-55 | 20-50 |
| *Composition III* | | | |
| Propylene glycol | 20-75 | 25-70 | 30-65 |
| Propylene glycol FAE** | 9-60 | 12-65 | 15-50 |
| Propylene carbonate | 3-50 | 5-45 | 7-40 |
| *Composition IV* | | | |
| Propylene glycol | | | |
| Propylene glycol FAE | 30-70 | 35-65 | 40-60 |
| N-methylpyrrolidone | 15-45 | 20-40 | 25-35 |
| *Composition V* | | | |
| Propylene glycol | 35-65 | 40-60 | 45-55 |
| Propylene glycol FAE | 9-31 | 12-28 | 15-25 |
| Propylene carbonate | 9-31 | 12-28 | 15-25 |
| N-methylpyrrolidone | 3-21 | 5-18 | 7-15 |

| | | | |
|---|---|---|---|
| *Theoretical composition of vehicle assuming final volume is equal to sum of volume of individual components **Propylene glycol fatty acid ester e.g. propylene glycol monocaprylate | | | |

In another aspect, the invention provides for the use of high concentrations of N-methylpyrrolidone as a non-aqueous vehicle for intranasal drug delivery. By high concentration, we mean that the N-methylpyrrolidone content of the vehicle in which the drug is dissolved is from about 40 to 100% by volume, more preferably from about 45 to 100% and most preferably from about 50 to 100%. The remainder of the vehicle will comprise other pharmaceutically-acceptable solvents (alone or in combination) and/or ingredients such as antioxidants, chelating agents, preservatives, flavourings, sweeteners or other agents generally used in pharmaceutical liquid preparations and well known to those skilled in the art. This vehicle is referred to hereinafter as the "N-methyl pyrrolidone vehicle".

Examples of pharmaceutically-acceptable solvents that may be used in combination with N-methyl pyrrolidone may be found in reference books such as the Handbook of Pharmaceutical Excipients (Fifth Edition, Pharmaceutical Press, London and American Pharmacists Association, Washington, 2006) and include, but are not limited to, propylene glycol, propylene carbonate, polyethylene glycol, ethanol, glycerol, glycofurol and propylene glycol fatty acid esters. However, a suitable composition may comprise a drug and N-methylpyrrolidone only, with no other ingredients added.

The use of this liquid vehicle will be discussed below with reference to use in combination with benzodiazepine drugs. However, this is by way of example only and this vehicle may also be used with other drugs such as those listed earlier in this text.

In a particular aspect, the present invention provides compositions suitable for intranasal delivery which comprise the N-methyl pyrrolidone vehicle and a benzodiazepine, such as those listed earlier herein, for example diazepam, lorazepam or midazolam.

The compositions comprising the N-methyl pyrrolidone vehicle may be prepared by dissolving the drug in the vehicle. The compositions comprising the N-methyl pyrrolidone vehicle preferably comprise from about 0.1 to about 2000 mg/ml, more preferably from about 0.5 to about 1500 mg/ml and most preferably from about 1 to about 1000 mg/ml of the drug.

If the drug is a benzodiazepine, the compositions comprising the N-methylpyrrolidone vehicle preferably comprise from about 0.1 to about 1000 mg/ml, more preferably from about 0.5 to about 800 mg/ml and most preferably from about 1 to about 600 mg/ml of the drug. For example, the preferred midazolam concentration from about 1 to about 400 mg/ml and the preferred lorazepam concentration is from about 0.5 to about 200 mg/ml.

A particularly preferred composition of the invention comprises the N-methyl pyrrolidone vehicle and diazepam. In this composition, the concentration of diazepam is preferably from about 1 to about 1000 mg/ml, more preferably from about 10 to about 800 mg/ml and most preferably from about 20 to about 600 mg/ml.

There are a number of different methods by which the drug formulations described in this application can be produced. For example, in one method the non-aqueous vehicle is first prepared by mixing together the vehicle components in the required quantities by volume or by weight. The required amount of drug and any other ingredients such as stabilisers or flavourings may then be weighed into a suitable vessel, a portion of the vehicle added (e.g. 90% of final amount) and the mixture stirred until the drug is dissolved. The drug solution is then made up to the required weight or volume by adding more of the drug to the non-aqueous vehicle. In another method, the drug (and any other ingredients if appropriate) is weighed into a suitable vessel and the exact weight of each solvent added. The mixture is then stirred until drug is dissolved. Following either of these methods, the final drug solution may be filtered if necessary.

Solutions comprising a vehicle of the invention and a drug may be administered to the nasal cavity in any suitable form for example in the form of drops or as a spray. The preferred method of administration is as a spray, e.g. using a spray device. Spray devices can be single ("unit") dose or multiple dose systems, for example comprising a bottle, pump and actuator, and are available from various commercial sources, including Pfeiffer (Germany), Valois (France), Rexam (France) and Becton-Dickinson (USA).

The present invention provides a nasal drug delivery device or a dose cartridge for use in a nasal delivery device loaded with a composition of the invention.

Nasal spray devices of the types described above typically dispense between 0.04 and 0.14 ml in a single actuation.

Typical nasal dosing regimens range from a single spray into one nostril to up to two sprays into each nostril.

The total liquid volume of solution delivered into the nasal cavity in one administration using the compositions of this invention is preferably from about 0.005 to about 1.0 ml, more preferably from about 0.01 to about 0.8 ml and most preferably from about 0.02 to about 0.6 ml, for example from about 0.1 to about 0.4 ml.

The present invention provides the use of a vehicle of the invention as described above in the manufacture of a medicament for the intranasal delivery of a drug to a patient in need of that drug.

The present invention provides compositions for use in the nasal delivery of a drug to a patient in need of that drug which compositions comprise a vehicle of the invention as described above and the drug.

The present invention provides processes for preparing the compositions of the invention. These processes are as described above.

The compositions of the invention comprising a benzodiazepine, such as those mentioned above, can be used to treat and/or prevent certain disorders, conditions or diseases of the central nervous system and in particular can be used to cause sedation, hypnosis, decreased anxiety, muscle relaxation, anterograde amnesia and anticonvulsant actions. They can also be used to treat epilepsy, insomnia, alcohol dependence, muscular disorders and mania. Thus, the present invention provides a method of administering a benzodiazepine drug compound, particularly a compound as listed above, to a patient in need thereof, for example for the prevention and/or treatment of the disorders, conditions or diseases set out above and/or to induce the effects set out above, which comprises the intranasal administration of a composition of the invention.

As used herein, we use the term patient to refer to both human and non-human animals. The invention is particularly suitable for use in the treatment of humans and animals such as dogs.

The present invention also provides the use of vehicle of the invention as described above and a benzodiazepine drug, such as a drug as listed above, in the manufacture of a medicament for nasal administration to a patient in need thereof. Such a medicament may be for the treatment and/or prevention of disorders, conditions or diseases of the central nervous system and/or to induce sedation, hypnosis, decreased anxiety, muscle relaxation, anterograde amnesia and anticonvulsant actions or treat epilepsy, insomnia, alcohol dependence, muscular disorders and mania.

The present invention also provides compositions comprising a vehicle of the invention as described above and a benzodiazepine drug compound and optionally additional ingredients as defined above for use in nasal delivery for treating disorders, conditions or diseases of the central nervous system and/or to induce sedation, hypnosis, decreased anxiety, muscle relaxation, anterograde amnesia and anticonvulsant actions or treat epilepsy, insomnia, alcohol dependence, muscular disorders and mania.

The invention is illustrated by the following non-limiting Examples.

### Example 1. Solution containing 50 mg/ml diazepam in propylene glycol/propylene carbonate (3:1)

The non-aqueous vehicle was prepared by mixing together 16.5 ml of propylene glycol (Sigma, Poole, UK) and 5.5 ml of propylene carbonate (Lyondell Chemical Co, USA) in a glass vial. 1 g of diazepam (Cambrex, Italy) was weighed into a 20 ml volumetric flask and 18 ml of the non-aqueous vehicle added. The flask contents were mixed using a magnetic stirrer and stirrer bar. When the drug had dissolved the stirrer bar was removed and the flask contents made up to volume using the non-aqueous vehicle.

### Example 2. Solution containing 50 mg/ml diazepam in propylene glycol/propylene glycol monocaprylate/propylene carbonate (5:4:1)

The non-aqueous vehicle was prepared by mixing together 11 ml of propylene glycol, 8.8 ml of propylene glycol monocaprylate (Capmul® PG-8, Abitec, USA) and 2.2 ml of propylene carbonate in a glass vial. 1 g of diazepam was weighed into a 20 ml volumetric flask and 18 ml of the non-aqueous vehicle added. The flask contents were mixed using a magnetic stirrer and stirrer bar. When the drug had dissolved the stirrer bar was removed and the flask contents made up to volume using the non-aqueous vehicle.

### Example 3. Solution containing 50 mg/ml diazepam in propylene glycol/propylene glycol monocaprylate/propylene carbonate (6:3:1)

The non-aqueous vehicle was prepared by mixing together 13.2 ml of propylene glycol, 6.6 ml of propylene glycol monocaprylate and 2.2 ml of propylene carbonate in a glass vial. 1 g of diazepam was weighed into a 20 ml volumetric flask and 18 ml of the non-aqueous vehicle added. The flask contents were mixed using a magnetic stirrer and stirrer bar. When the drug had dissolved the stirrer bar was removed and the flask contents made up to volume using the non-aqueous vehicle.

### Example 4. Solution containing 50 mg/ml diazepam in propylene glycol/propylene glycol monocaprylate/propylene carbonate (4.5:4.5:1)

The non-aqueous vehicle was prepared by mixing together 9.9 ml of propylene glycol, 9.9 ml of propylene glycol monocaprylate and 2.2 ml of propylene carbonate in a glass vial. 1 g of diazepam was weighed into a 20 ml volumetric flask and 18 ml of the non-aqueous vehicle added. The flask contents were mixed using a magnetic stirrer and stirrer bar. When the drug had dissolved the stirrer bar was removed and the flask contents made up to volume using the non-aqueous vehicle.

### Example 5. Solution containing 50 mg/ml diazepam in propylene glycol/propylene glycol monoeaprylate/N-methylpyrrolidone (5:3:2)

The non-aqueous vehicle was prepared by mixing together 11.0 ml of propylene glycol, 6.6 ml of propylene glycol monocaprylate and 4.4 ml of N-methylpyrrolidone (Sigma) in a glass vial. 1 g of diazepam was weighed into a 20 ml volumetric flask and 18 ml of the non-aqueous vehicle added. The flask contents were mixed using a magnetic stirrer and stirrer bar. When the drug had dissolved the stirrer bar was removed and the flask contents made up to volume using the non-aqueous vehicle.

### Example 6. Solution containing 50 mg/ml diazepam in propylene glycol/propylene glycol monocaprylate/propylene carbonate (6:2:2)

The non-aqueous vehicle was prepared by mixing together 9.9 ml of propylene glycol, 9.9 ml of propylene glycol monocaprylate and 2.2 ml of propylene carbonate in a glass vial. 1 g of diazepam was weighed into a 20 ml volumetric flask and 18 ml of the non-aqueous vehicle added. The flask contents were mixed using a magnetic stirrer and stirrer bar. When the drug had dissolved the stirrer bar was removed and the flask contents made up to volume using the non-aqueous vehicle.

### Example 7. Solution containing 75 mg/ml diazepam in propylene glycol/propylene glycol monocaprylate/propylene carbonate (5:3.5:1.5)

The non-aqueous vehicle was prepared by mixing together 11 ml of propylene glycol, 7.7 ml of propylene glycol monocaprylate and 3.3 ml of propylene carbonate in a glass vial. 1.5 g of diazepam was weighed into a 20 ml volumetric flask and 18 ml of the non-aqueous vehicle added. The flask contents were mixed using a magnetic stirrer and stirrer bar. When the drug had dissolved the stirrer bar was removed and the flask contents made up to volume using the non-aqueous vehicle.

### Example 8. Solution containing 75 mg/ml diazepam in propylene glycol/propylene carbonate (1:1)

The non-aqueous vehicle was prepared by mixing together 11 ml of propylene glycol and 11 ml of propylene carbonate in a glass vial. 1.5 g of diazepam was weighed into a 20 ml volumetric flask and 18 ml of the non-aqueous vehicle added. The flask contents were mixed using a magnetic stirrer and stirrer bar. When the drug had dissolved the stirrer bar was removed and the flask contents made up to volume using the non-aqueous vehicle.

### Example 9. Solution containing 75 mg/ml diazepam in propylene glycol/propylene glycol monocaprylate/N-methylpyrrolidone/propylene carbonate (5:2:1:2)

The non-aqueous vehicle was prepared by mixing together 11 ml of propylene glycol, 4.4 ml of propylene glycol monocaprylate, 4.4 ml of propylene carbonate and 2.2 ml of N-methylpyrrolidone in a glass vial. 1.5 g of diazepam was weighed into a 20 ml volumetric flask and 18 ml of the non-aqueous vehicle added. The flask contents were mixed using a magnetic stirrer and stirrer bar. When the drug had dissolved the stirrer bar was removed and the flask contents made up to volume using the non-aqueous vehicle.

### Example 10. Solution containing 125 mg/ml diazepam in propylene glycol/propylene glycol monocaprylate/propylene carbonate (1:1:1)

The non-aqueous vehicle was prepared by mixing together 7.33 ml of propylene glycol, 7.33 ml of propylene glycol monocaprylate and 7.33 ml of propylene carbonate in a glass vial. 1.5 g of diazepam was weighed into a 20 ml volumetric flask and 18 ml of the non-aqueous vehicle added. The flask contents were mixed using a magnetic stirrer and stirrer bar. When the drug had dissolved the stirrer bar was removed and the flask contents made up to volume using the non-aqueous vehicle.

### Example 11. Single dose nasal spray delivering 5 mg of diazepam

For any of Examples 1 to 6, 0.125 ml of solution was dispensed into the glass vial of a Pfeiffer (Radolfzell, Germany) unit dose spray device. The vial is sealed with an elastomer closure, placed into the vial holder and the vial holder snapped into place onto the actuator piece of the spray device. On actuation, the device will dispense 0.1 ml of liquid as a spray plume and containing 5 mg of diazepam.

### Example 12. Multiple dose nasal spray delivering 7.5 mg of diazepam

For any of Examples 7 to 9, 2 ml of solution is dispensed into a 3 ml "U-Save" glass vial (SGD Pharma, France). A Valois nasal spray pump (0.1 ml spray volume) is screwed onto the vial. The spray pump is primed by actuating four times. Each actuation of the primed pump will dispense 0.1 ml of liquid as a spray plume and containing 7.5 mg of diazepam. Each bottle will dispense approximately 15 x 7.5 mg doses of diazepam.

### Example 13. Solution containing 20 mg/ml midazolam in propylene glycol/propylene glycol monocaprylate/propylene carbonate (2:1:1)

The non-aqueous vehicle was prepared by mixing together 4 ml of propylene glycol, 2 ml of propylene glycol monocaprylate and 2 ml of propylene carbonate in a glass vial. 100 mg of midazolam (Sifa, Ireland) was weighed into a 5 ml volumetric flask and 4 ml of the non-aqueous vehicle was added. The flask contents were stirred until the drug had dissolved and the solution was made up to volume with the non-aqueous vehicle.

### Example 14. Solution containing 10 mg/ml lorazepam in propylene glycol/propylene glycol monocaprylate/propylene carbonate (3:1:1)

The non-aqueous vehicle was prepared by mixing together 3 ml of propylene glycol, 1 ml of propylene glycol monocaprylate and 1 ml of propylene carbonate in a glass vial. 20 mg of lorazepam (Sigma) was weighed into a second glass vial and 2 ml of the non-aqueous vehicle added. The vial contents were stirred until the drug had dissolved.

### Example 15. Solution containing 10 mg/ml lorazepam in propylene glycol/N-methylpyrrolidone (1:1)

The non-aqueous vehicle was prepared by mixing together 3 ml of propylene glycol and 3 ml of N-methylpyrrolidone in a glass vial. 20 mg of lorazepam (Sigma) was weighed into a second glass vial and 2 ml of the non-aqueous vehicle added. The vial contents were stirred until the drug had dissolved.

### Example 16. Solution containing 200 mg/ml diazepam in N-methylpyrrolidone

1 gram of diazepam was weighed into a volumetric flask. Approximately 4 ml of N-methylpyrrolidone was added and the flask contents stirred until the drug was dissolved. The flask contents were then made up to volume with N-methylpyrrolidone.

## Claims

1. A composition for intranasal delivery of a drug comprising:
(i) the drug; and
(ii) a non-aqueous vehicle comprising (a) propylene glycol and at least one additional solvent selected from N-methylpyrrolidone, propylene carbonate and at least one propylene glycol fatty acid ester, or (b) from about 40 to 100 % by volume of N-methylpyrrolidone.

2. A composition according to claim 1, wherein the drug is a benzodiazepine.

3. A composition according to claim 2, wherein the drug is selected from alprazolam, chlordiazepoxide, clonazepam, clorazepate, diazepam, estazolam, flurazepam, halazepam, lorazepam, midazolam, nitrazepam, oxazepam, prazepam, quazepam, temazapem, bromazepam, flunitrazepam and triazolam, bentazepam, brotizolam, clotiazepam, delorazepam, ethyl loflazepate, etizolam, fludiazepam, ketozolam, loprazolam, lormetazepam, nordazepam, mexazolam, nimetazepam, pinazepam and tetrazepam.

4. A composition according to claim 3, wherein the drug is diazepam, lorazepam or midazolam.

5. A composition according to any one of the preceding claims comprising a vehicle (a) in which the additional solvent is or comprises at least one propylene glycol fatty acid ester.

6. A composition according to claim 5, wherein the or each propylene glycol fatty acid ester is propylene glycol monocaprylate.

7. A composition according to any one of the preceding claims comprising a vehicle (a) in which the additional solvent is or comprises propylene carbonate.

8. The use of a non-aqueous composition comprising (a) propylene glycol and at least one additional solvent selected from N-methylpyrrolidone, propylene carbonate and at least one propylene glycol fatty acid ester, or (b) from about 40 to 100 % by volume of N-methylpyrrolidone as a vehicle for the intranasal delivery of a drug.

9. Use according to claim 8, wherein the drug is a benzodiazepine.

10. Use according to claim 9, wherein the drug is selected from alprazolam, chlordiazepoxide, clonazepam, clorazepate, diazepam, estazolam, flurazepam, halazepam, lorazepam, midazolam, nitrazepam, oxazepam, prazepam, quazepam, temazapem, bromazepam, flunitrazepam and triazolam, bentazepam, brotizolam, clotiazepam, delorazepam, ethyl loflazepate, etizolam, fludiazepam, ketozolam, loprazolam, lormetazepam, nordazepam, mexazolam, nimetazepam, pinazepam and tetrazepam.

11. Use according to claim 10, wherein the drug is diazepam, lorazepam or midazolam.

12. A nasal drug delivery device or a dose cartridge for use in a nasal drug delivery device loaded with a composition as defined in any one of claims 1 to 7.

13. The use of a non-aqueous vehicle comprising (a) propylene glycol and at least one additional solvent selected from N-methylpyrrolidone, propylene carbonate and at least one propylene glycol fatty acid ester, or (b) from about 40 to 100 % by volume of N-methylpyrrolidone in the manufacture of a medicament for the nasal administration of a drug to a patient in need thereof.

14. Use according to claim 13, wherein the drug is a benzodiazepine.

15. Use according to claim 14, wherein the drug is selected from alprazolam, chlordiazepoxide, clonazepam, clorazepate, diazepam, estazolam, flurazepam, halazepam, lorazepam, midazolam, nitrazepam, oxazepam, prazepam, quazepam, temazapem, bromazepam, flunitrazepam and triazolam, bentazepam, brotizolam, clotiazepam, delorazepam, ethyl loflazepate, etizolam, fludiazepam, ketozolam, loprazolam, lormetazepam, nordazepam, mexazolam, nimetazepam, pinazepam and tetrazepam.

16. Use according to claim 15, wherein the drug is diazepam, lorazepam or midazolam.

17. The use of a benzodiazepine drug and a non-aqueous vehicle comprising (a) propylene glycol and at least one additional solvent selected from N-methylpyrrolidone, propylene carbonate and propylene glycol fatty acid esters, or (b) from about 40 to 100 % by volume of N-methylpyrrolidone in the manufacture of a medicament for the treatment and/or prevention of disorders, conditions or diseases of the central nervous system.

18. Use according to claim 17, in the manufacture of a medicament for inducing sedation, hypnosis, decreased anxiety, muscle relaxation, anterograde amnesia or anticonvulsant actions.

19. Use according to claim 18, in the manufacture of a medicament for the treatment and/or prevention of epilepsy, insomnia, alcohol dependence, muscular disorders or mania.

20. A composition comprising a benzodiazepine drug and a non-aqueous vehicle comprising (a) propylene glycol and at least one additional solvent selected from N-methylpyrrolidone, propylene carbonate and propylene glycol fatty acid esters, or (b) from about 40 to 100 % by volume of N-methylpyrrolidone, for use in intranasal administration to treat and/or prevent disorders, conditions or diseases of the central nervous system.

21. A composition comprising a benzodiazepine drug and a non-aqueous vehicle comprising (a) propylene glycol and at least one additional solvent selected from N-methylpyrrolidone, propylene carbonate and propylene glycol fatty acid esters, or (b) from about 40 to 100 % by volume of N-methylpyrrolidone, for use in intranasal administration to induce sedation, hypnosis, decreased anxiety, muscle relaxation, anterograde amnesia or anticonvulsant actions.

22. A composition comprising a benzodiazepine drug and a non-aqueous vehicle comprising (a) propylene glycol and at least one additional solvent selected from N-methylpyrrolidone, propylene carbonate and propylene glycol fatty acid esters, or (b) from about 40 to 100 % by volume of N-methylpyrrolidone, for use in intranasal administration to treat and/or prevent epilepsy, insomnia, alcohol dependence, muscular disorders or mania.

23. A method of administering a benzodiazepine drug to a patient in need thereof, which method comprises the intranasal administration of a composition as defined in any one of claims 1 to 7.

24. A method of treating and/or preventing disorders, conditions or diseases of the central nervous system, which method comprises the intranasal administration of a composition as defined in any one of claims 1 to 7.

25. A method of inducing sedation, hypnosis, decreased anxiety, muscle relaxation, anterograde amnesia or anticonvulsant actions, which method comprises the intranasal administration of a composition as defined in any one of claims 1 to 7.

26. A method of treating or preventing epilepsy, insomnia, alcohol dependence, muscular disorders or mania, which method comprises the intranasal administration of a composition as defined in any one of claims 1 to 7.
